# EUROPÄISCHE PATENTSCHRIFT

(11) **EP 0 435 063 B1**
(45) Veröffentlichungstag und Bekanntmachung des Hinweises auf die Patenterteilung: **24.08.1994**
(21) Anmeldenummer: 90123831.1
(22) Anmeldetag: 11.12.1990
(51) Int. Cl.: A61M 11/06, A61M 3/00

(54) **Naseninjektionsvorrichtung**
Device for nasal injection
Dispositif pour injection nasale

(30) Priorität: 23.12.1989 DE 8915166 U
(43) Veröffentlichungstag der Anmeldung: 03.07.1991
(73) Patentinhaber: Strahl, H. Michael, Dr. Med., D-40545 Düsseldorf (DE)
(72) Erfinder: Strahl, H. Michael, Dr. Med., D-40545 Düsseldorf (DE)
(74) Vertreter: Stenger, Watzke & Ring Patentanwälte

(56) Entgegenhaltungen:
- AT-B- 774
- DE-U- 8 809 117
- US-A- 2 283 668
- US-A- 2 887 106

## Beschreibung

Die Erfindung betrifft eine Vorrichtung zum Injizieren von Flüssigkeiten in die Nase, wie beispielsweise Nasenspülungen mit Medikamenten, Salzlösungen oder Ölen, mit einer in die Nase einzuführenden Kanüle, die an ihrem proximalen Ende eine sich in Richtung des distalen Endes der Kanüle erstreckende glockenartige Kappe hat, welche eine zentrale Öffnung mit einem Durchmesser aufweist, der mit dem Durchmesser der Kanüle übereinstimmt, wobei der an die Kanüle angrenzende Kappenrand dicker ausgebildet ist als der dem distalen Kanülenende zugewandte Kappenrand.

Aus der US-A-2 887 106 ist eine gattungsbildende Vorrichtung bekannt. Bei dieser, den nasalen Teil eines Inhalationsapparates bildenden Vorrichtung ist es nicht notwendig, daß sich diese genau der Form des Nasenloches anpaßt, da sie nur zum Einleiten heißer Dämpfe in die Nase dient. Bei einer erfindungsgemäßen Spülvorrichtung ist es jedoch wichtig, daß die Vorrichtung druckdicht mit der Naseninnenwand abschließt.

Aus der DE-U-88 09 117 ist ferner bekannt, bei einer Nasenspülvorrichtung ein in die Nase einzuführendes Teil aus einer Silikonabformmasse herzustellen. Durch dieses nasale Teil führt ein Schlauch, durch den der Patient die Spülflüssigkeit ansaugen kann. Zwar ist es auch möglich, den Schlauch auf eine Spritze aufzustecken, jedoch bedarf es jeweils einer besonderen, an den Durchmesser des Schlauches angepaßten Spritze, was einer einfachen und unproblematischen Handhabung dieser bekannten Vorrichtung entgegenwirkt.

Weiterhin ist eine Vorrichtung bekannt, die ein Glasröhrchen umfaßt, dessen erstes Ende eine kleine Öffnung aufweist und an dessen zweiten Ende ein Gummibalg angeordnet ist, mit dem die in die Nase einzuträufelnde Flüssigkeit in das Röhrchen eingesaugt wird. Es ist ferner eine Vorrichtung bekannt, die vorzugsweise für die Injektion von gelartigen, schleimhautabschwellenden Medikamenten in die Nase verwendet wird. Diese Vorrichtung besteht aus einem vorzugsweise als Fläschchen ausgebildeten Behälter, der mit einem schraubbaren Verschluß verschlossen ist. In der Mitte des Verschlusses ist ein Röhrchen angeordnet, das mit einem einen Ende bis in die im Behälter befindliche Flüssigkeit reicht und mit seinem anderen Ende durch den Verschluß greift. Auf dem Röhrchen ist axial verschiebbar eine in die Nase einzuführende Kanüle angeordnet. Diese Kanüle weist an ihrem proximalen Teil eine kleine Öffnung auf. An der Kanüle ist weiterhin ein umlaufender Fingeransatz angeordnet, mit dem die Kanüle in axialer Richtung zum Verschluß hin gedrückt wird, so daß in dem Behälter ein Überdruck entsteht und das Medikament aus der Öffnung in die Nase gespritzt wird.

Bei allen diesen Vorrichtungen ist es nachteilig, daß das Nasenloch bei der Injektion des Medikamentes nicht vollständig abgeschlossen und das Medikament auch durch Einatmen nur unzureichend bis in die oberen Bereiche der Nase befördert wird. Weiterhin ist es nachteilig, daß diese Vorrichtungen nicht für die Anwendung verschiedener Medikamente geeignet sind.

Ausgehend von diesem Stand der Technik liegt der Erfindung die **Aufgabe** zugrunde, eine Vorrichtung zum Injizieren von Flüssigkeiten in die Nase zu schaffen, die einfach anwendbar ist und sich selbsttätig der Form eines Nasenlochs so anpaßt, daß sie das Nasenloch vollständig abschließt.

Die **Lösung** dieser Aufgabenstellung durch die Erfindung ist dadurch gekennzeichnet, daß die Kappe und die Kanüle aus einem elastischen Werkstoff bestehen und daß die Kanüle ein elastisches distales Ende aufweist, mit dem sie über eine Öffnung eines die Flüssigkeit enthaltenen Behälters gestülpt wird, insbesondere über den Konus einer Kolbenspritze.

Bei dieser erfindungsgemäßen Ausgestaltung wird die Anpassungsfähigkeit der in das Nasenloch einzuführenden Kanüle verbessert und gleichzeitig eine bessere Abdichtung zwischen der Kappe und der Nase erzielt; ferner ist es für den Anwender angenehmer, eine elastische Kanüle in das Nasenloch einzuführen.

Aufgrund des elastischen distalen Endes der Kanüle kann diese in einfacher Weise über eine Öffnung eines die Flüssigkeit bzw. das Medikament enthaltenden Behälters gestülpt werden. Vorzugsweise werden hierzu Kolbenspritzen mit verschiedenen Volumina benutzt, so daß die Medikamentendosis an den Patienten und die Therapie angepaßt werden kann.

Durch die am proximalen Ende der Kanüle angeordnete glockenartige Kappe wird das Nasenloch bei der Einführung der Kanüle vollständig abgeschlossen, so daß das Medikament bei der Injektion nicht wieder aus dem Nasenloch herausläuft. Dies ist insbesondere dann vorteilhaft, wenn extrem dünnflüssige Medikamente verwendet werden, wie beispielsweise Salzlösungen. Durch die glockenartige Ausgestaltung der Kappe paßt sich die Vorrichtung dem Nasenloch selbsttätig an. Wenn der Durchmesser der in der Kappe angeordneten Öffnung mit dem Durchmesser der Kanüle übereinstimmt, kann die zu injizierende Flüssigkeit ungehindert aus der Kanüle austreten und es kommt nicht zu Medikamentenablagerungen an Übergangsstellen zwischen der Kanüle und der Kappe.

Zur einfacheren Handhabung und zur Erzielung einer preiswerten Ausgestaltung der Vorrichtung ist es vorteilhaft, die Kanüle und die Kappe einteilig auszubilden.

Bei einer bevorzugten Ausführungsform der erfindungsgemäßen Vorrichtung ist in dem proximalen Ende der Kanüle eine Rückstaudüse angeordnet, die ausgangsseitig perforiert ausgebildet ist. Hierdurch wird eine gute Verteilung der Flüssigkeit im Nasenloch durch ein Verspritzen der Flüssigkeit erzielt.

Es ist schließlich vorteilhaft, wenn die Rückstaudüse drehbar in der Kanüle angeordnet ist und zwischen einer Sprühstellung und einer Spritzstellung verstellt werden kann. Durch diese Ausgestaltung sind gezielte Anwendungen möglich, die verschiedene Therapien in Abhängigkeit der erwünschten Heilerfolge und der benutzten Flüssigkeiten zulassen.

Weitere Einzelheiten und Vorteile ergeben sich aus der nachfolgenden Beschreibung der zugehörigen Zeichnung, in welcher eine bevorzugte Ausführungsform einer erfindungsgemäß ausgebildeten Vorrichtung zum Injizieren von Flüssigkeiten in die Nase dargestellt ist. In der Zeichnung zeigen:
- Fig. 1: eine Vorrichtung zum Injizieren von Flüssigkeiten in die Nase;
- Fig. 2: eine Vorrichtung gemäß Fig. 1 mit einer Rückstaudüse und
- Fig. 3: eine Vorrichtung gemäß Fig. 2, die auf eine handelsübliche Kolbenspritze aufgesetzt ist.

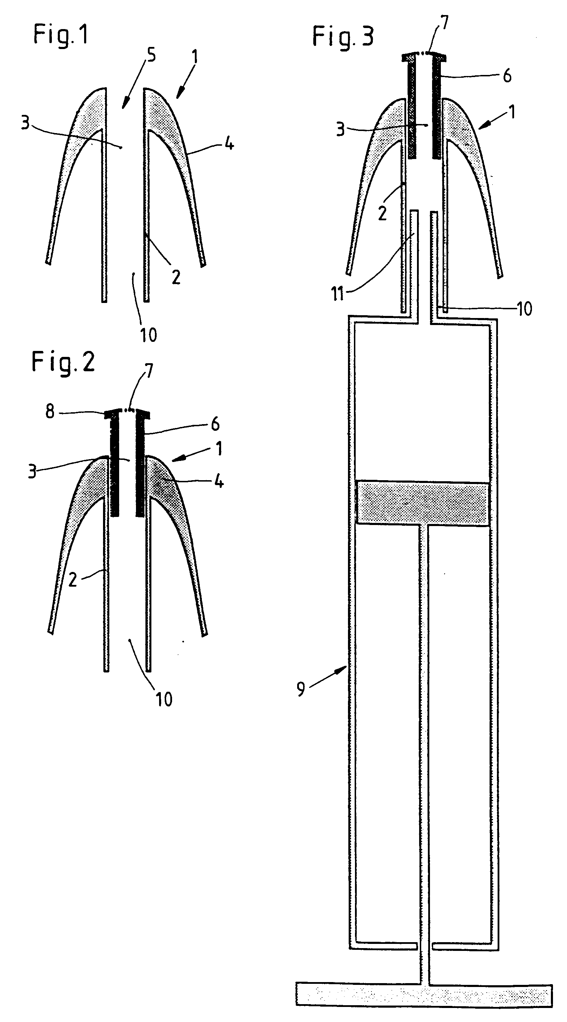

In Fig. 1 ist mit der Bezugsziffer 1 eine erfindungsgemäße Vorrichtung zum Injizieren von Flüssigkeiten in die Nase bezeichnet. Die Vorrichtung 1 besteht aus einer Kanüle 2 und einer am proximalen Ende 3 der Kanüle 2 angeordneten Kappe 4. Die Kappe 4 hat eine Öffnung 5 mit einem Durchmesser, der dem Durchmesser der Kanüle 2 entspricht.

Die Kappe 4 weist eine glockenartige, annähernd konische Form auf, wobei der am proximalen Ende 3 der Kanüle 2 anliegende Kappenrand dicker ausgebildet ist als der dem proximalen Ende 3 der Kanüle 2 abgewandte Kappenrand.

Die Fig. 2 zeigt eine erfindungsgemäße Vorrichtung 1, bei der in das proximale Ende 3 der Kanüle 2 eine Rückstaudüse 6 eingesetzt ist. Die Rückstaudüse 6 besteht aus einem Rohrstück, das an seinem einen Ende mit einer perforierten Platte 7 abschließt. Die Rückstaudüse 6 weist weiterhin an diesem Ende eine umlaufende Kante 8 auf, mit der sich die Rückstaudüse 6 im eingesetzten Zustand auf der Kappe 4 abstützt.

Die Fig. 3 zeigt die Vorrichtung zum Injizieren von Flüssigkeiten in die Nase in Kombination mit einer handelsüblichen Kolbenspritze 9. Die Vorrichtung 1 ist mit ihrem distalen Ende 10 auf den Konus 11 der Kolbenspritze 9 aufgesteckt. In das proximale Ende 3 der Kanüle 2 ist wiederum die Rückstaudüse 6 mit der perforierten Platte 7 eingesetzt.

### Bezugszeichenliste:

- 1: Vorrichtung zum Injizieren
- 2: Kanüle
- 3: proximales Ende
- 4: Kappe
- 5: Öffnung
- 6: Rückstaudüse
- 7: perforierte Platte
- 8: Kante
- 9: Kolbenspiegel
- 10: distales Ende
- 11: Konus

## Patentansprüche

1. Vorrichtung zum Injizieren von Flüssigkeiten in die Nase, wie beispielsweise Nasenspülungen mit Medikamenten, Salzlösungen oder Ölen, mit einer in die Nase einzuführenden Kanüle (2), die an ihrem proximalen Ende (3) eine sich in Richtung des distalen Endes (10) der Kanüle (2) erstreckende glockenartige Kappe (4) hat, welche eine zentrale Öffnung (5) mit einem Durchmesser aufweist, der mit dem Durchmesser der Kanüle (2) übereinstimmt, wobei der an die Kanüle (2) angrenzende Kappenrand dicker ausgebildet ist als der dem distalen Kanülenende (10) zugewandte Kappenrand,
**dadurch gekennzeichnet,**
daß die Kappe (4) und die Kanüle (2) aus einem elastischen Werkstoff bestehen und
daß die Kanüle (2) ein elastisches distales Ende (10) aufweist mit dem sie über eine Öffnung eines die Flüssigkeit enthaltenden Behälters gestülpt wird, insbesondere über den Konus (11) einer Kolbenspritze (9).

2. Vorrichtung nach Anspruch 1, dadurch gekennzeichnet, daß die Kanüle (2) und die Kappe (4) einteilig ausgebildet sind.

3. Vorrichtung nach einem der Ansprüche 1 oder 2, dadurch gekennzeichnet, daß in dem proximalen Ende (3) der Kanüle (2) eine Rückstaudüse (6) angeordnet ist, die ausgangsseitig eine perforierte Platte (7) aufweist.

4. Vorrichtung nach Anspruch 3, dadurch gekennzeichnet, daß die Rückstaudüse (6) drehbar in der Kanüle (2) angeordnet ist und zwischen einer Sprühstellung und eine Spritzstellung verstellt werden kann.

## Claims

1. Device for injecting liquids into the nose, such as, for example, nasal irrigations with medicaments, saline solutions or oils, having a cannula (2) which is to be introduced into the nose and which has, at its proximal end (3), a bell-shaped cap (4) extending in the direction of the distal end (10) of the cannula (2), which cap (4) has a central opening (5) with a diameter which corresponds to the diameter of the cannula (2), the cap edge adjoining the cannula (2) being designed thicker than the cap edge facing towards the distal cannula end (10), characterized in that the cap (4) and the cannula (2) consist of elastic material, and in that the cannula (2) has an elastic distal end (10) with which it can be pushed over an opening of a receptacle containing the liquid, in particular over the cone (11) of a plunger syringe (9).

2. Device according to Claim 1, characterized in that the cannula (2) and the cap (4) are designed in one piece.

3. Device according to either of Claims 1 and 2, characterized in that there is arranged, in the proximal end (3) of the cannula (2), a backpressure nozzle (6) which has, at the output side, a perforated plate (7).

4. Device according to Claim 3, characterized in that the backpressure nozzle (6) is arranged rotatably in the cannula (2) and can be adjusted between a spray position and an injection position.

## Revendications

1. Dispositif pour injecter des liquides dans le nez, comme par exemple des injections nasales contenant des médicaments, des solutions salines ou des huiles, ledit dispositif comprenant une canule (2) qui doit être introduite dans le nez et qui comporte, à son extrémité proximale (3), une coiffe (4) en forme de cloche qui s'étend en direction de l'extrémité distale (10) de la canule (2) et qui comporte une ouverture centrale (5) présentant un diamètre coïncidant avec le diamètre de la canule (2), le bord de la coiffe adjacent à la canule (2) étant plus épais que le bord de la coiffe tourné vers l'extrémité distale (10) de la canule, caractérisé en ce que la coiffe (4) et la canule (2) sont réalisées dans un matériau élastique, et en ce que la canule (2) comporte une extrémité distale élastique (10) qui permet de l'emmancher sur l'ouverture d'un réservoir contenant le liquide, en particulier sur le cône (11) d'une seringue à piston (9).

2. Dispositif selon la revendication 1, caractérisé en ce que la canule (2) et la coiffe (4) sont réalisées d'une seule pièce.

3. Dispositif selon l'une des revendications 1 ou 2, caractérisé en ce qu'il est prévu, dans l'extrémité proximale (3) de la canule (2), une buse de retenue (6) qui comporte une plaque perforée (7) sur le côté sortie.

4. Dispositif selon la revendication 3, caractérisé en ce que la buse de retenue (6) est disposée avec possibilité de rotation dans la canule (2) et peut être déplacée entre une position de pulvérisation et une position d'injection.
